# EUROPEAN PATENT SPECIFICATION

(11) **EP 2 911 710 B1**
(45) Date of publication and mention of the grant of the patent: **21.11.2018**
(21) Application number: 13792520.2
(22) Date of filing: 23.10.2013
(51) Int. Cl.: A61L 31/02, A61L 31/10, A61L 31/14

(54) **FULLY ABSORBABLE INTRALUMINAL DEVICES AND METHODS OF MANUFACTURING THE SAME**
VOLLSTÄNDIG ABSORBIERBARE INTRALUMINALE VORRICHTUNGEN UND VERFAHREN ZUR HERSTELLUNG DAVON
DISPOSITIFS INTRALUMINAUX TOTALEMENT ABSORBABLES ET LEURS PROCÉDÉS DE FABRICATION

(30) Priority: 23.10.2012 US 201261795695 P
(43) Date of publication of application: 02.09.2015
(73) Proprietor: Zorion Medical, Inc., Zionsville, IN 46077 (US)
(72) Inventor: STECKEL, Mark, Glasgow G12 9QP (GB); PANDELIDIS, Ioannis, O., Sharon, MA 02067 (US)
(74) Representative: HGF Limited
(86) International application number: PCT/US2013/066307
(87) International publication number: WO 2014/066465

(56) References cited:
- WO-A1-2008/034066
- US-A1- 2010 256 728
- US-A1- 2011 238 149
- US-A1- 2011 319 977
- US-B1- 7 105 018

## Description

### TECHNICAL FIELD

The present teachings are generally related to intraluminal devices, and more particularly to fully absorbable intraluminal implants.

### BACKGROUND

The field of coronary angioplasty and stenting has made dramatic progress in the treatment of coronary heart disease through at least three generations of product technology. As these generational advancements are achieved, however, new challenges or failure modes of therapy are also typically present. For instance, while balloon angioplasty therapy improved acute luminal flow, associated vessel recoil and remodeling resulted in high restenosis rates. Bare metal stenting, on the other hand, has been found to lower restenosis rates and minimize abrupt closure events, however restenosis rates were still high due to stent mechanical injury and resulting smooth muscle cell (SMC) migration and proliferation into the lumen. While drug eluting stents can significantly cut the retreatment rate by addressing the SMC proliferation with a pharmaceutical agent, such stents are known to suffer from problems associated with late stent thrombosis (LST), as well as the extended use of anti-coagulants. LST is associated with high mortality rates, although the frequency of the events is relatively low. The apparent factors driving this serious complication appear to be the loss of vaso-motion and delayed healing of a functional endothelium.

In peripheral arteries, the use of conventional stents have had limited success due to high restenosis rates, long term fracture of struts, and concerns about limiting re-intervention options due to the difficulty in re-wiring occluded small distal vessels that have been previously stented with conventional non-absorbable metal stents. Whereas drug coated balloons have had some success in peripheral arteries, this technology suffers from very short drug delivery times (typically 1 to 2 minutes) and arterial dissections that require bare metal stenting to stabilize occlusive flaps.

Attempts to use magnesium and its alloys as a temporary implant biomaterial, including in cardiovascular stents, have been hindered by poor control over the rate and uniformity of the metal's degradation (metallic corrosion rate), fragmentation and absorption processes in local tissue. Previous attempts to control degradation or corrosion rates have focused on alloying with rare earth and other heavy metal elements of unknown biocompatibility, yielding slower metallic corrosion rates but unproven benefits in clinical performance. Although these approaches have merit for non-medical applications such as commercial or aerospace castings, they are sub-optimal for an absorbable implant grade material that will eventually be fully metabolized by the host tissue, and release alloying elements of unknown biocompatibility. Furthermore, conventional approaches to corrosion control of magnesium alloys have focused solely on preventing the initial mechanical failure of the given article by retarding the degradation process either by a surface passivation layer, or changing the local corrosion potential of the alloy. No consideration has been given to controlling the process of fragmentation, disintegration and absorption following the initial mechanical failure. For many implant applications, the timing and nature of the full degradation process, starting with the as-implanted metal article to the final clearance of the alloy mass and its degradants from the anatomical site, is critical regarding the performance of the medical device.

One such implant application is absorbable metal stents for vascular or luminal scaffolding, such as stents for treatment of coronary artery disease. In this application, the stents provide a temporary scaffolding through the healing process related to the local injury caused by the high pressure angioplasty balloon used to open the partially blocked artery. The metal scaffold is typically required only for a period of days to weeks to prevent abrupt closure of the vessel from spasm, to minimize elastic recoil, and to serve as a substrate to deliver a controlled release drug-polymer formulation to the site of injury. After this period, any remnant of the alloy or its conversion products may be a liability, since it can act as a foreign body prolonging an inflammatory response and delaying healing. Furthermore, if the stent remnants remain present in the lumen in solid form through the period of extracellular matrix deposition and scar formation, then the stent remnants themselves become a source of lumen obstruction and participate in a new form of restenosis unknown to conventional permanent stents.

An alternative design approach towards absorbable stents utilizes highly crystalline absorbable polymers such as poly-L-lactide (PLLA) for the structural elements of the stent scaffold. This approach has a more controlled degradation process, however suffers from low radial stiffness that is needed to open the artery (i.e., so-called acute gain, and limited ductility making stent-artery sizing problematic, especially for tapered vessels). Furthermore, the lengthy absorption times of 2 years and longer for the crystalline domains of PLLA has unknown effects on the duration of anti-platelet therapy that is required to prevent life-threatening blood clots.

The current standard of care for treating most *de novo* coronary lesions is the implantation of a permanent implant known as a drug eluting stent or DES. The DES is a third generation angioplasty device for treating coronary and peripheral stenosis, with significantly lower re-intervention rates than either bare metal stents or balloon angioplasty. This generation technology is a permanent implant, typically comprising a high strength and high radiopacity metal such as cobalt chrome or platinum enriched stainless steel, coated with a formulation of an anti-proliferative drug in a controlled release polymer.

The next generation of technology for vascular disease is a fully absorbable stent (or fully absorbable drug eluting stent), i.e. the entire mechanical scaffolding (stent) and the drug carrier formulation is broken down in the body and absorbed. The working hypothesis is that any permanent foreign body at the site can prolong inflammation and delay healing and restoration to its native state. One major complication that fully absorbable stents should address is late stent thrombosis, which is believed to result from this delayed healing and permanent inhibition of vaso-motion with conventional stented vessels. Another benefit of fully absorbable stents is that they can serve as a temporary platform for extended drug delivery in small peripheral vessels, without causing a potential limitation to future endo-vascular re-interventions.

The primary focus of fully absorbable (also sometimes referred to as 'bioabsorbable' and 'resorbable') stents has been on achieving the necessary hoop strength and stiffness to bear the high mechanical stresses presented in the given physiologic environment, whether coronary arteries, peripheral arterial or venous structures, or larger lumens such as the aorta, esophagus, or trachea. A second key characteristic that is required is radio-opacity to enable the physician to visualize the stent after implantation by x-ray. Since the two primary materials used in experimental absorbable stents, L-poly lactic acid and magnesium alloys, are both essentially radio-transparent, it is advantageous to include discrete radio-markers typically at the ends of the stent comprised of platinum, platinum-iridium, or tantalum for x-ray visualization. Even though the discrete radio-marker features are bio-stable and no not absorb, such structures are generally considered to be fully absorbable stents.

The present teachings are intended to improve upon and resolve some of these known deficiencies of the art.

US 2011/319977 A1 describes a bioabsorbable implant including an elongated metallic element including more than 50% a metal substantially free of rare earth metals.

US 2002/0165601 A1 describes a preferably self-expanded stent of the woven or braided type made entirely of bioabsorbable material, the stent is either totally or partially covered by a film of a bioabsorbable material that can conform to the stent deformation.

US 2010/256728 A1 describes biodegradable stent grafts and methods for treatment of early stage aneurysms and acute vessel injuries. The stent frameworks themselves are a biodegradable metal and the graft material is a semi-permeable, biodegradable porous fabric.

US 7 105 018 B1 describes an intravascular stent including an eluting sheath fabricated from a mesh for controlled release of therapeutic drugs and for delivery of the therapeutic drugs in localized drug therapy in a blood vessel.

### SUMMARY

In accordance with one aspect of the present teachings, a fully absorbable intraluminal device is provided and comprises a helical tubular magnesium alloy structure including a series of continuous and longitudinally uncoupled sinusoidal, planar waveform segments, the tubular magnesium alloy structure having a polymer surface coating and being substantially free of rare earth metals; and an expandable polymeric mesh sleeve at least partially bonded to the polymer surface coating, the expandable polymeric mesh sleeve configured to form a mechanical coupling with the magnesium alloy structure, wherein the expandable polymeric mesh sleeve includes a plurality of apertures that are configured to provide strain relief and improve expandability of the sleeve.

In an example, the absorbable intraluminal device comprises a magnesium alloy wire form having a polymer surface coating, the wire form comprising more than 50% by weight of one or more metals
selected from magnesium, iron, zinc, calcium and manganese and being substantially free of rare earth metals; and an expandable polymeric mesh sleeve at least partially bonded to the polymer surface coating to form a mechanical coupling with the magnesium alloy wire form, the sleeve comprising a linear polyester high polymer selected from one or more of poly lactic acid, polyglycolic acid, polydioxanone, polytrimethylenecarbonate and copolymers and blends thereof. In accordance with this specific embodiment, the device is fully absorbable within 30 to 365 days after being implanted into a luminal body, yet able to maintain a structural integrity sufficient for acutely holding the luminal body open.

In accordance with yet another aspect of the present teachings, a method of fabricating a fully absorbable intraluminal device is provided. According to this illustrative embodiment, the method comprises forming a wire into a radially expandable tubular wire form having a series of continuous and physically uncoupled sinusoidal, planar waveform segments, the wire form comprising more than 50% by weight of one or more metals selected from magnesium, iron, zinc, calcium and manganese and being substantially free of rare earth metals; coating the formed tubular wire form with a polymer surface coating having a linear polyester high polymer selected from one or more of polylactic acid, polyglycolic acid, polydioxanone, polytrimethylenecarbonate and copolymers and blends thereof; and bonding at least a portion of an expandable polymeric mesh sleeve to the polymer surface coating to form a mechanical coupling with the tubular wire form, the expandable polymeric mesh sleeve having a surface with a plurality of apertures that are configured to provide strain relief and improve expandability of the sleeve. In this embodiment, the device is fully absorbable, yet able to maintain a structural integrity sufficient for acutely holding a luminal body open.

Other objects and benefits of the teachings will become apparent from the following written description along with the accompanying figures.

### BRIEF DESCRIPTION OF THE DRAWINGS

The above-mentioned aspects of the present teachings and the manner of obtaining them will become more apparent and the teachings will be better understood by reference to the following description of the embodiments of the teachings taken in conjunction with the accompanying drawings, wherein:
FIG. 1 is a schematic diagram illustrating a wire formed into a continuous sinusoidal-like wave form in accordance with the teachings of the present disclosure;
FIG. 2a is a schematic diagram of the wire of FIG. 1 formed into a helical tubular structure and consisting of a series of continuous and longitudinally uncoupled sinusoidal, planar waveform segments in accordance with the teachings of the present disclosure;
FIG. 2b is a schematic diagram of the helical tubular wire structure of FIG. 2a shown in an expanded state;
FIG. 3a is a top view of an expandable polymeric mesh material in accordance with the teachings of the present disclosure;
FIG. 3b is a perspective view of the expandable polymeric mesh material of FIG. 3a formed into a tubular sleeve in accordance with the teachings of the present disclosure;
FIG. 3c is a perspective view of the polymeric mesh tubular sleeve of FIG. 3b shown in an expanded state in accordance with the teachings of the present disclosure;
FIG. 4a is a perspective view of the expandable polymeric mesh tubular sleeve of FIG. 3b partially bonded to an external surface of a polymer surface coating of the helical tubular wire structure of FIG. 2a in accordance with the teachings of the present disclosure;
FIG. 4b is a partial perspective view of the bonded polymeric mesh tubular sleeve and helical tubular wire structure of FIG. 4a shown in an expanded state in accordance with the teachings of the present disclosure;
FIG. 4c is a perspective cross-sectional view of the expanded polymeric mesh tubular sleeve and helical tubular wire structure of FIG. 4b;
FIG. 5 is a perspective view of the expandable polymeric mesh tubular sleeve of FIG. 3b partially bonded to an internal surface of a polymer surface coating of the helical tubular wire structure of FIG. 2a in accordance with the teachings of the present disclosure;
FIG. 6 is a perspective view of the helical tubular wire structure of FIG. 2a sandwiched between two expandable polymeric mesh tubular sleeves in accordance with the teachings of the present disclosure; and
FIG. 7 is a perspective cross-sectional view of the sandwiched helical tubular wire structure of FIG. 6.

### DETAILED DESCRIPTION

In the following detailed description, reference is made to the accompanying drawings, which form a part hereof. In the drawings, similar symbols typically identify similar components, unless context dictates otherwise.

Unless defined otherwise, all technical and scientific terms used herein have the same meaning as commonly understood by one of ordinary skill in the art to which this present teachings belong. Although any method and materials similar or equivalent to those described herein can be used in the practice or testing of the present teachings, the specific methods and materials are now described. Moreover, the techniques employed or contemplated herein are standard methodologies well known to one of ordinary skill in the art and the materials, methods and examples are illustrative only.

The present teachings are generally directed to intraluminal medical devices that, in accordance with certain embodiments, can be implanted into a luminal structure by a catheter-based delivery system. In accordance with certain specific embodiments, the target lumen could be an arterial or venous vascular structure, a segment of the gastro-intestinal tract including the esophagus, a segment of the urinary or reproductive tracts, an airway passage including the trachea, a segment of the biliary duct or any other hollow vessel where therapeutic treatment necessitates either temporary structural support and or local drug delivery to the luminal surface.

According to certain aspects of the present disclosure, the intraluminal devices utilize a unique hybrid design that combines absorbable metal features with that of absorbable polymer structural features. Unlike conventional absorbable stents that do not utilize such a platform, the present hybrid design offers radial strength and a broad range of expandability through its metallic properties, as well as axial or longitudinal stability and increased surface area coverage through its polymeric structural features. Current absorbable stents are typically either 100% polymer based systems, which do not provide optimal radial strength or a broad range of expansion, or 100% absorbable metal based systems, that are difficult to manufacture and do not have optimum mechanical properties due to limited ductility.

To achieve the desired properties associated with this hybrid design, the present teachings utilize a high purity, absorbable metal wire that is formed into a helical, continuous sinusoid that is physically bonded to an expandable polymeric tubular mesh sleeve. Such a system creates a stent like structure that possesses the short term requisite radial and axial mechanical behavior desired for an intraluminal implant, yet still permits the structure to rapidly and safely break down and therefore become fully absorbed by the body within a time frame that is desirable for soft tissue healing. In accordance with certain embodiments herein, the intraluminal implants of the present disclosure are fully absorbable within about 30 to about 365 days, and more particularly within about 120 to about 270 days, after being implanted into a luminal body. Prior to full absorption, the implants are able to maintain a structural integrity that is sufficient for holding the luminal body open.

While those of skill in the art should understand and appreciate herein that a broad range of absorbable materials may be utilized to form the absorbable implants in accordance with the teachings of the present disclosure, according to one specific embodiment herein, the wire form component is formed from a high purity magnesium based alloy, while the expandable polymeric mesh sleeve component is formed from various polymers or co-polymers based on polylactic acid or polyglycolic acid and their derivatives. As will be more fully explained below, the mechanism used to mechanically couple these two components to one another allows the system to maintain its structural integrity during the implantation and acute healing processes. To achieve this desired mechanical coupling, in accordance with certain aspects of the present disclosure, the magnesium based wire form is coated with a compatible absorbable polymer that is thermally bonded to the expandable polymer mesh during the manufacturing process.

As those of skill will readily understand and appreciate herein, processes for manufacturing conventional, non-absorbable stents using a helical, continuous sinusoidal wire form are well known in the art. For instance, materials such as 316L stainless steel and cobalt chromium are routinely processed into planar sinusoids and then helically wound onto a mandrel or shaft to form a cylindrical stent like structure. Despite the availability of such manufacturing techniques, helical, continuous sinusoidal wire forms that are fabricated in accordance with conventional techniques are insufficient for functioning as intra-luminal stents, particularly as such wire forms are known to longitudinally unravel when expanded and/or their rings separated *in vivo* when subjected to physiologic loading stresses. Because of the inherent structural deficiencies encountered by these conventional wire forms, axial connectors are often incorporated between the rings of the wire in order to add mechanical integrity and strength. For conventional non-absorbable metal wire form based stents, this is typically achieved by spot welding adjacent rings through a laser or resistive welding process. These processes, however, are highly problematic for absorbable metal wire forms (such as magnesium based alloy systems); particularly as the magnesium surfaces rapidly form oxide layers that in turn inhibit strong metal to metal bonds from being formed. Welding of fine magnesium structures is further complicated by the material's intrinsic high thermal conductivity, such that heat energy applied to the local weld area is rapidly dissipated to the entire structure. In addition, even if a mechanical bond could be formed, the welding zone significantly changes the microstructure of the magnesium based alloy, thereby resulting in local embrittlement, undesirable axial stiffness, and non-uniform biodegradation rates.

While some conventional systems have combined magnesium alloy stents with absorbable polymer coatings in order to slow down the *in vivo* oxidation rate of the magnesium (as a carrier for a drug) or to purportedly mitigate local pH effects of the magnesium degradation, these systems have not done so as a means to achieve structural integrity of the magnesium based wire form stent or to significantly increase surface area coverage of the expanded stent by providing axial connections.

Increased surface area coverage of an absorbable intraluminal implant is highly advantageous for some indications, and particularly when it is desired to trap the native plaque or thrombus between the implant and the vessel wall, as opposed to having it prolapse through the interstices of the expanded stent and thereby released downstream (resulting in obstructions of distal vessels). As will be demonstrated in more detail below, unlike typical wire form or laser cut absorbable stents, which cover approximately 15% of the vessel surface area when expanded, the embodiments of the present disclosure are unexpectedly capable of covering greater than 50% of the luminal surface area, while increasing the available surface area for drug delivery and lowering focal stresses on the native vessel wall by distributing expansive forces over a larger surface area. More particularly, the fully absorbable intraluminal devices of the present teachings are capable of achieving a device-to-luminal surface area coverage of greater than 50%.

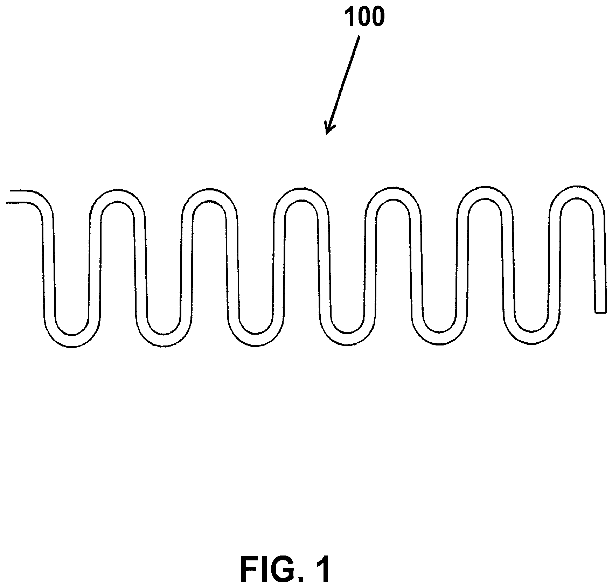

Moving now to FIG. 1, a schematic diagram illustrating a wire 100 formed into a continuous sinusoidal-like wave form in accordance with the teachings of the present disclosure is shown. In accordance with this illustrative embodiment, the wire 100 is formed from an absorbable metal component or alloy. While the absorbable metal components used to form the wire 100 in accordance with the present teachings can be fabricated from a variety of absorbable metallic materials, in accordance with certain aspects, the metal components include pure and alloyed metals that are capable of oxidizing in physiologic environments in order to achieve full breakdown and absorption over a period of time sufficient for soft tissue healing. Illustrative metal components that may be used in accordance with the present teachings include, but are not limited to, pure metals and alloys of magnesium, zinc and iron, and particularly alloys that are substantially free of rare earth metals. As used herein, the term "substantially free of rare earth metals" is intended to mean that less than 500 ppm of the metallic alloy includes rare earth metals. To this end, it should be understood that the metallic alloy components of the present teachings should have a high purity and fine gran size (i.e., less than 20 microns) in order to achieve consistent strength and *in vivo* degradation rates in thin walled struts regardless of the alloy. As those of skill in the art will understand and appreciate herein, keeping the metallic alloy components substantially free of rare earth metals will allow the component to be naturally absorbed by the body, and particularly such that its structural integrity will not be negatively impacted by the inherently corrosive properties of the rare earth metals.

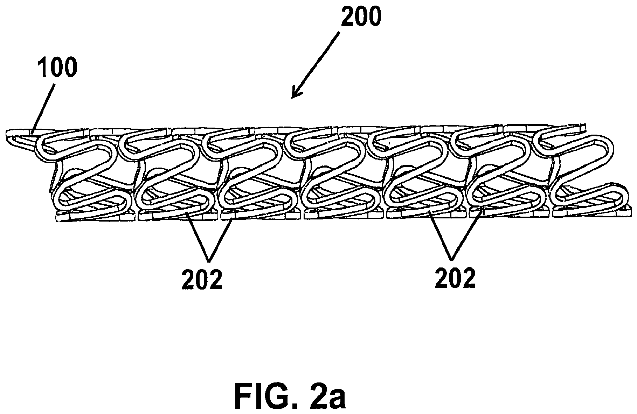

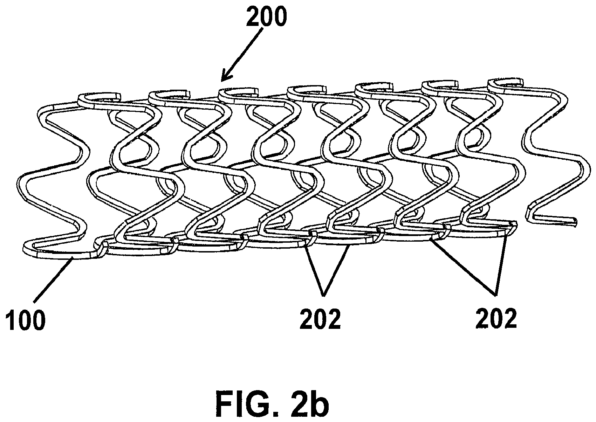

For magnesium based absorbable metals of the present disclosure, either pure magnesium or high purity alloys that contain one or more of lithium, calcium, manganese, zinc, iron, aluminum or combinations thereof may be used. In accordance with certain aspects of the present teachings, the alloy wire form 100 may be comprised of more than 50% by weight of one or more metals selected from magnesium, iron, zinc, calcium and manganese. In accordance with still other aspects of the present teachings in which alloys of magnesium are used to form the alloy wire form 100, the magnesium alloy contains between about 1% and about 25% by weight lithium. Whatever specific components are used to form the alloy wire form 100, as is shown in FIGS. 2a and 2b, the resulting alloy wire form should be formable into a helical tubular structure 200 consisting of a series of continuous and longitudinally uncoupled sinusoidal, planar waveform segments 202 (i.e., the structure does not have axial connectors between the rings of the formed wire). In accordance with certain aspects of the present disclosure in which a magnesium based alloy wire form is utilized, the wire form may have a strut thickness between about 50 microns and about 150 microns.

Various wire forming methods are generally known within the art, and as such, the fabrication methods envisioned by the present teachings are not intended to be limited herein. In accordance with certain aspects of the present disclosure, the helical tubular structure 200 is formed by a die drawing method (that helps to achieve reduction in cross-sectional diameter) together with an in process thermal annealing operation (to offset work hardening). According to certain aspects herein, the wire 100 can be processed by conventional wire forming methods that utilize a rotating pin table or a table of fixed pins. In addition, if desired, the final wire form can be electro-polished to remove surface contaminants, as well as to reduce its final diameter. Moreover, while not required herein, in accordance with certain aspects of the present teachings, it may also be beneficial to smelt the metallic alloys under vacuum and in pyrolitic carbon molds in order to minimize impurities.

As mentioned above, the helical tubular structures 200 of the present disclosure offer radial strength and a broad range of expandability through their metallic properties. An illustrative depiction of these beneficial properties can be appreciated from FIG. 2b, which specifically shows the helical tubular wire structure 200 in an expanded state.

In addition to the expandability of the disclosed helical tubular wire structure 200, in accordance with certain aspects of the present disclosure, the wire structure further comprises a polymeric surface coating selected from a synthetic or natural absorbable polymeric component. In accordance with this aspect of the present teachings, the polymer surface coating may include synthetic and natural polymers selected from, but not limited to, aliphatic and cyclic polyesters, polyanhydrides, polycarbonates, and polypeptides such as collagen, elastin or gelatin. One particularly useful class of absorbable polymers that can be used in accordance with the present teachings are synthetic linear polyesters, particularly because of their mechanical properties and established clinical uses and biocompatibility, as well as their ability to process-by melt (extrusion) or solvent (spray coating) methods. These polymers may be synthesized from a variety of monomers such as lactic acid (PLA), glycolic acid (PGA), caprolactone (PCL), diaxanone (PDO), and other close derivatives. These monomers may also be combined during polymerization to form co-polymers (i.e. PLGA is a copoplymer of PLA and PGA), with relative fractions controlled to influence crystallinity, degradation rate, and thermal stability. Likewise, polymers based on two or more monomer types may be physically blended to achieve improved elasticity or altered absorption rate. In accordance with certain aspects of the present disclosure, the polymer surface coating is comprised of a linear polyester high polymer selected from one or more of polylactic acid, polyglycolic acid, polydioxanone, polytrimethylenecarbonate and copolymers and blends thereof.

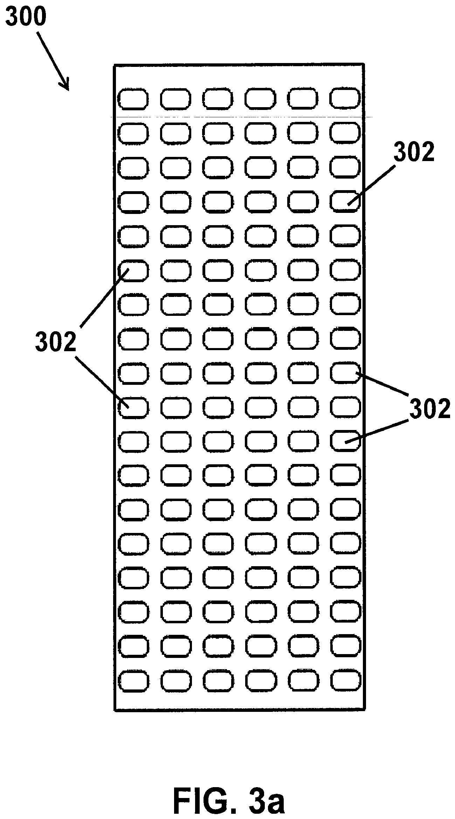

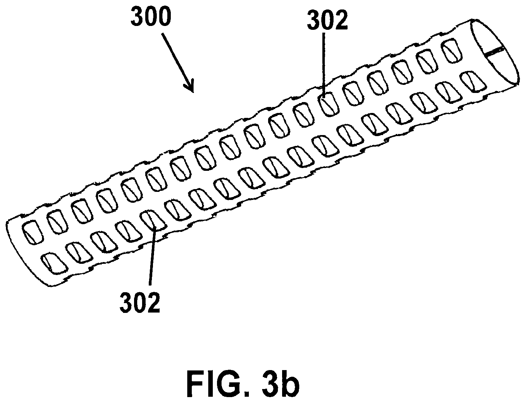

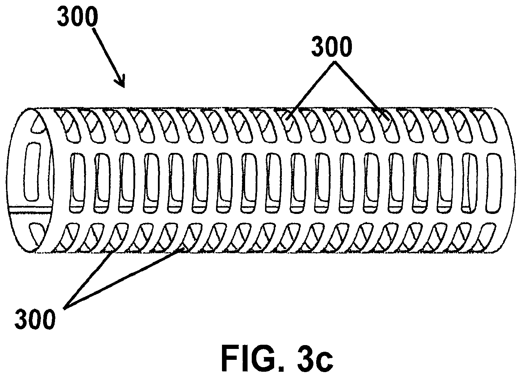

Moving now to FIGS. 3a-3c, an expandable polymeric mesh sleeve 300 that is at least partially bondable to the polymer surface coating of the helical tubular wire structure 200 is shown. In particular, FIG. 3a is a top view of the expandable polymeric mesh sleeve 300, while FIG. 3b is a perspective view of the expandable polymeric mesh material formed into a tubular sleeve in accordance with the teachings of the present disclosure. Finally, FIG. 3c is a perspective view of the polymeric mesh tubular sleeve 300 shown in an expanded state.

Much like the polymer surface coating of the wire structure 200, a broad range of known absorbable biomaterials can be utilized to form the polymeric mesh tubular sleeve 300 in accordance with the present teachings. These absorbable biomaterials include all of the synthetic and natural polymers listed above and useable to form the polymeric surface coating of the wire structure. In terms of synthetic aliphatic polyesters (such as the family of PLGA homopolymers and copolymers) that may be used in accordance with the teachings of the present disclosure, it should be understood and appreciated herein that several important variables can be controlled in order to achieve a functional and fully absorbable implant. For instance, in accordance with certain illustrative embodiments, the molecular weight may be greater than about 40000 Da so that the component may be fully processed as well as exhibit extended strength retention *in vivo.* Moreover, the polymer can be thoroughly dried before thermal processing (e.g., extrusion) in order to minimize molecular weight loss due to hydrolysis. In addition, the polymer can have a low initial monomer level (less than 1 %) and be of high purity. The polymer can be extruded by conventional methods to a thin walled tube (typically less than 25 microns wall thickness) or dissolved in a solvent such as acetone and cast into a tube or a thin coating (typically less than 5 microns) on a wire form. Alternatively, both the polymer and a drug can be dissolved in a solvent like acetone or ethyl acetate and can be spray coated or ink jet coated to the external surface of the intraluminal implant to form a fully absorbable drug eluting stent.

In accordance with certain aspects herein, the intraluminal implants formed according to the present disclosure may be configured to function with one or more discrete, non-absorbable radio-opaque features and/or comprise one or more therapeutic agents. As those of skill in the art will understand and appreciate herein, various therapeutic agents that may be used with the presently disclosed fully absorbable intraluminal implants include, but are not limited to, anti-restenotic agents, anti-stenotic agents, antiproliferative agents, immunomodulators, antithrombotics, antioxidants, estrogen, growth factor inhibitors, antisense oligonucleotides, collagen inhibitors, chemotherapeutic agents and combinations thereof. In addition, the therapeutic agents can be one or more drugs selected from one or more of paclitaxel and related taxanes, rapamycin, sirolimus, everolimus, tacrolimus, heparin and benzalkonium heparinate.

Still referring to FIGS. 3a-3c, in accordance with certain aspects of the present teachings, the expandable polymeric mesh sleeves 300 may be subjected to further processing steps in order to improve their expandability. For instance, according to certain embodiments, the polymeric mesh sleeves 300 may be processed by laser cutting or other means to form longitudinal slots (patterns) 302 or apertures which provide strain relief and improve the expandability of the component. The apertures could be created by several conventional methods, including, but not limited to, fluid or air jet, or by direct molding or casting in a tool.

In accordance with certain aspects of the present disclosure, the polymeric mesh sleeves 300 can be formed into heat shrink tubes to aid in the hybrid stent assembly by expanding the tubes between about 10 and about 40% of the extruded diameter at a temperature between its glass transition temperature and melting point (e.g., about 60°C for PLA) and then rapidly cooling the tube to freeze in the oriented micro-structure. After assembling the expanded tube over the wire form 200, it may be heated to above about 60°C to allow the tube to shrink tightly onto the metal wire form.

Advantages and improvements of the processes, methods and devices of the present invention are demonstrated in the following examples.

### Example 1:

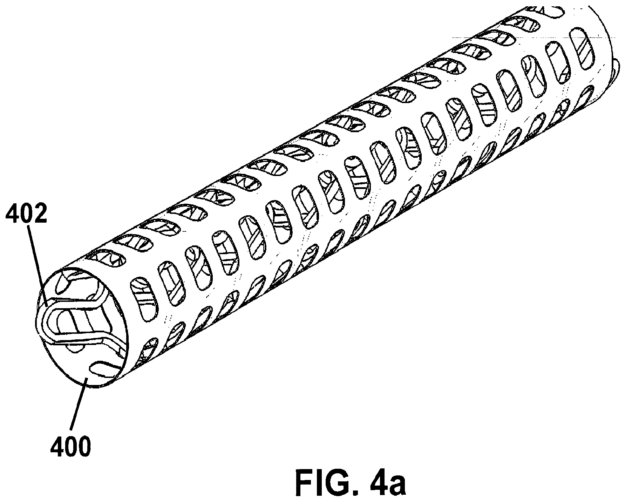

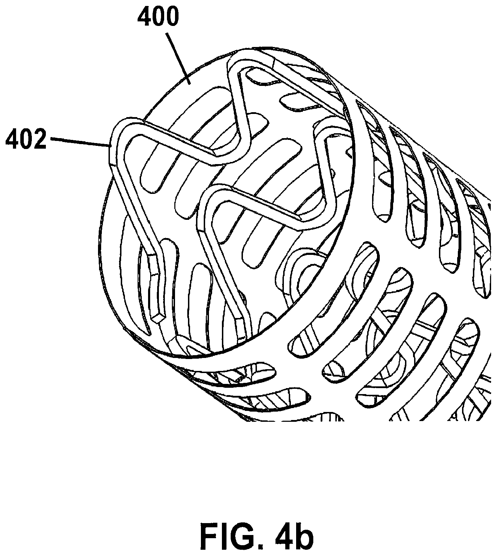

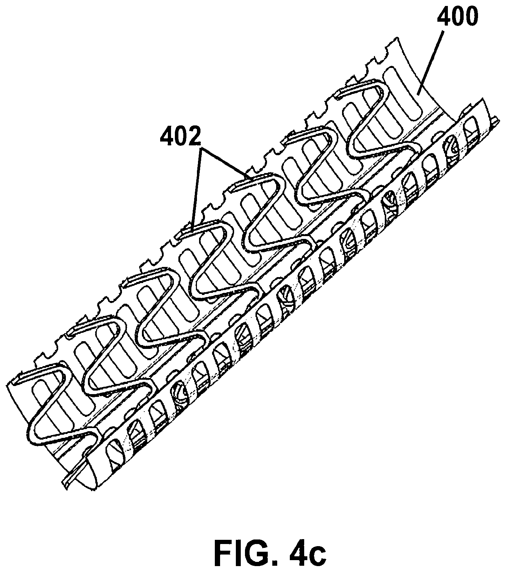

A high purity alloy of Mg-4%Li rod was cast and was subsequently drawn into fine wire of 125 microns in diameter by conventional cold drawing through progressively smaller dies with intermediate annealing treatments. The wire was then formed into a sinusoidal waveform (amplitude of 1.5mm) on a manual bending jig around a 350 micron diameter pin. The continuous sinusoid was coiled around 1.05 mm shaft and annealed, forming a cylindrical Mg-Li stent wire form of 12mm in length. The Mg-Li wire form was cleaned in isopropanol, oven dried, and then spray coated with a PGLA 50-50 copolymer (Mn=70000 Da). Separately, a 1.3 mm diameter tube was extruded from PLLA and was laser cut with longitudinal slots to enable expansion. The 14 mm long slotted PLLA tube was then slightly expanded by heating to 60°C and stretched over a tapered mandrel of 1.8 mm inner diameter and cooled. The hybrid stent was then assembled by sliding the PLLA extruded tubular sheath over the Mg-Li wireform and then heated to 70°C causing the PLLA tube to shrink onto and to stick to the PGLA coated surface of the Mg-Li wire form (see for Example FIG. 4a-4c, which show an illustrative expandable polymeric mesh tubular sleeve 400 partially bonded to an external surface of a polymer surface coating of a helical tubular wire structure 402). The coated stent was crimped on a 3.5mm balloon angioplasty catheter stent delivery system using a conventional iris mechanical crimper, sterilized by ethylene oxide gas, and packaged in a foil package backfilled with nitrogen gas. The stent was balloon deployed into the iliac artery of a rabbit where it provided acute mechanical up to 7 days post implant. The expanded and deployed hybrid stent had a surface area coverage of 63% combined between wire form and polymer mesh.

### Example 2:

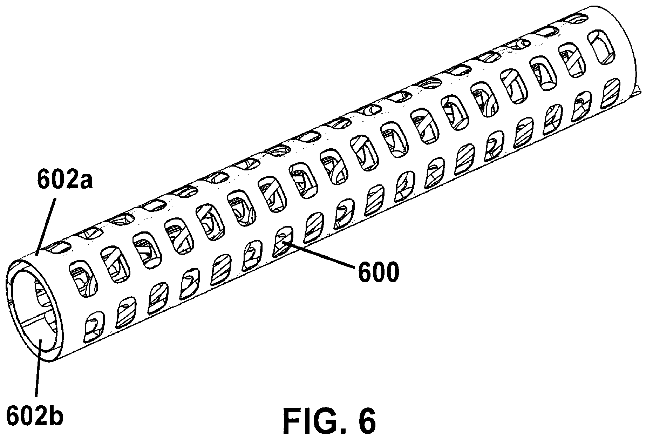

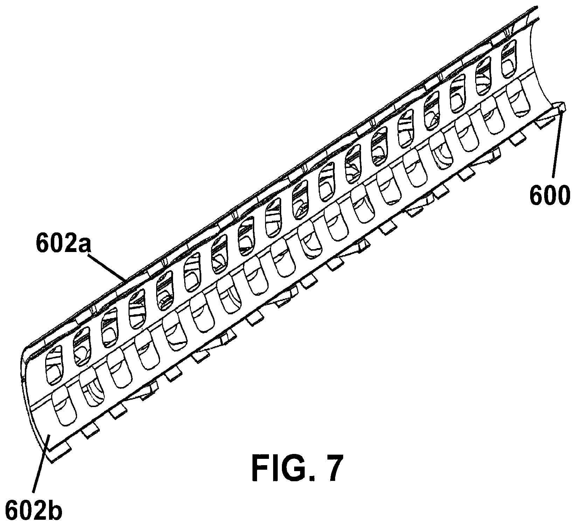

A hybrid fully absorbable stent identical to Example 1 is made with a Mg-Li wire form and 5050 PLGA coating, except that a 3-layer hybrid stent is formed with the Mg-Li wire form 'sandwiched' between an inner and outer layer of laser cut PLLA tubing (see for example FIGS. 6 and 7, which show an illustrative helical tubular wire structure 600 sandwiched between two expandable polymeric mesh tubular sleeves 602a, 602b). The assembly is manufactured in a silicone split cavity mold. The hybrid stent is assembled over a length of 1 mm OD silicone tubing, with the PLLA tubing loaded first, followed by the Mg-Li wire form, and then another, larger diameter (about 1.7mm) PLLA tubing. The mold with the entire assembly is placed in a vacuum oven at 65°C for 10 minutes with the mold clamped together, whereas the combination of heat and pressure result in the polymer within the 3 layers bonding together. The assembly is crimped on a 3.0 mm balloon catheter stent delivery system, packaged and sterilized. When expanded to its nominal diameter of 3.0 mm, the 3 layer structure forms a structure with greater than 80% surface area coverage, wherein the largest apertures through the wall of stent are on the order of 150µ and hence the structure is able to prevent typical thrombus or plaque from prolapsing through the stent and embolization.

### Example 3:

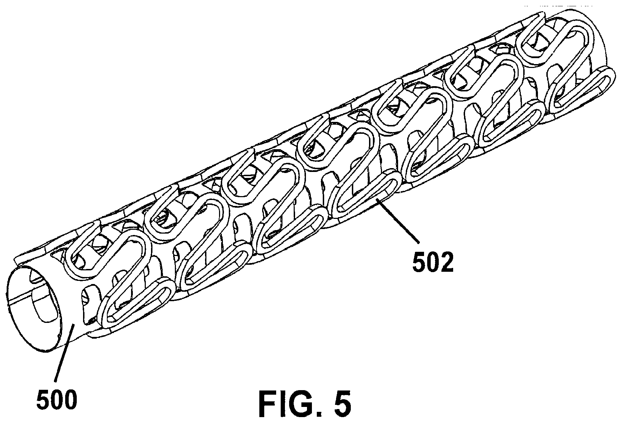

A fine grain, high purity Mg-1.0 Ca is cast and drawn into 95µ wire using conventional die drawing methods. The wire is formed into a continuous sinusoidal (1.0mm amplitude) on a programmable, automated wire forming machine around a hardened 0.27mm pin. The wire form is wound around a 0.9mm stainless steel shaft, and annealed, resulting in a cylindrical wire form of about 1.1 mm ID which is cleaned in acetone, air dried, then spray coated with a dilute solution of 50-50 PLGA and paclitaxel in methylene chloride solvent to a thickness of about 3-5µ. The formulation is a dry weight of 5% Paclitaxel and 95% PLGA and results in about 1 microgram of drug per mm stent length or less. Separately, a 90-10 PGLA tube is extruded with a 1.0 mm OD, and laser cut in a fine zig-zag mesh capable of expansion. The laser cut PLGA tube is then loaded on 0.9mm silicone tubing connected to an inflation device, and the Mg-Ca wire form is loaded over the PLGA tube (see for example FIG. 5, which shows an expandable polymeric mesh tubular sleeve 500 partially bonded to an internal surface of a polymer surface coating of a helical tubular wire structure 502). The silicone tubing is inflated with 80°C water to 1 atmosphere pressure for 5 minutes resulting in compaction and thermal fusing of the 2 layer structure together. The drug eluting fully absorbable stent is then crimped on a 4.0mm balloon delivery system using a temperature controlled iris crimper from Machine Solutions Inc., Utah USA. It is terminally packaged in a foil pack with a Tyvek header, vacuum dried and the Tyvek header removed in a final heat sealing operation.

### Example 4:

A high purity magnesium is cast, grain refined and drawn into 120µ hypotube. The hypotube is laser cut into individual (and distinct or uncoupled) zig zag rings of 1.0mm amplitude and electropolished to 100µ strut thickness. The rings are assembled in a mandrel with a DL-PLA laser cut sheath inner layer, the Mg rings spaced evenly over the length of the assembly, and an outer DL-PLA laser cut sheath. The assembly and mandrel is placed in a silicone rubber split mold and fused under vacuum at 70°C for 5 minutes. The outer surface sheath is coated with a 50-50 by weight Sirolimus-DL-PLA formulation applied by a fluid dispensing printer. The Sirolimus eluting fully absorbable stent is crimped on a balloon catheter, EtO sterilized and foil packed.

### Example 5:

A balloon expandable 30mm diameter fully absorbable drug eluting stent is made for local delivery of chemo-agents to esophageal lumen as part of a cancer therapy. The hybrid stent is formed from a Mg-06%Li (by weight) 160 micron drawn wire that is formed with a 5-axis robotic system around a table array of 0.50 mm diameter pins to form a continuous sinusoid of 3 mm in amplitude. The wire form is wrapped around a 9.0 mm mandrel to form a helical continuous sinusoid, electro-polished down to 150 micron strut thickness, spray-coated with a 6 micron thick coating of D,L-PLA. It is combined with an outer sheath that is solvent cast from D,L-PLA into a 30 micron thick wall tube. The tube is water jet cut in a mesh pattern to increase expandability. The polymer tube is expanded at 60°C to 11mm and cooled. The tube is slid over the coated wire form and heated to 70°C for 30 minutes under vacuum, whereas the tube shrinks tightly and thermally bonds to the coated surface of the wire form. The surface is coated with a therapeutic dose of a taxane chemo-agent, mounted on a 30 Fr balloon catheter and sterilized by ethylene oxide gas.

## Claims

1. A fully absorbable intraluminal device, comprising:
a helical tubular magnesium alloy structure (200) including a series of continuous and longitudinally uncoupled sinusoidal, planar waveform segments (202), the tubular magnesium alloy structure (200) having a polymer surface coating and being substantially free of rare earth metals; and
an expandable polymeric mesh sleeve (300) at least partially bonded to the polymer surface coating, the expandable polymeric mesh sleeve (300) configured to form a mechanical coupling with the magnesium alloy structure (200), wherein the expandable polymeric mesh sleeve (300) includes a plurality of apertures (302) that are configured to provide strain relief and improve expandability of the sleeve (300).

2. The fully absorbable intraluminal device of claim 1, wherein the magnesium alloy structure (200) comprises a magnesium alloy wire form.

3. The fully absorbable intraluminal device of claim 1, wherein the device is fully absorbable within 30 to 365 days after being implanted into a luminal body, yet able to maintain a structural integrity sufficient for acutely holding the luminal body open.

4. The fully absorbable intraluminal device of claim 1, wherein the magnesium alloy structure (200) has a strut thickness between 50 microns (µm) and 150 microns (µm).

5. The fully absorbable intraluminal device of claim 1, wherein the polymer surface coating is comprised of a linear polyester high polymer selected from one or more of polylactic acid, polyglycolic acid, polydioxanone, polytrimethylenecarbonate and copolymers and blends thereof;
or
wherein the expandable polymeric mesh sleeve (300) is comprised of a linear polyester high polymer selected from one or more of poly lactic acid, polyglycolic acid, polydioxanone, polytrimethylenecarbonate and copolymers and blends thereof.

6. The fully absorbable intraluminal device of claim 1, wherein the expandable polymeric mesh sleeve (300) is at least partially bondable to an internal surface of the polymer surface coating to form the indirect bond with the magnesium alloy structure (200);
or
wherein the expandable polymeric mesh sleeve (300) is at least partially bondable to an external surface of the polymer surface coating to form the indirect bond with the magnesium alloy structure (200).

7. The fully absorbable intraluminal device of claim 1, wherein the device is configured to function in conjunction with one or more discrete, non-absorbable radio-opaque features.

8. The fully absorbable intraluminal device of claim 1, further comprising one or more therapeutic agents selected from an anti-restenotic agent, an anti-stenotic agent, an antiproliferative agent, an immunomodulator, an antithrombotic, an antioxidant, estrogen, a growth factor inhibitor, an antisense oligonucleotide, a collagen inhibitor, a chemotherapeutic agent and combinations thereof;
wherein, optionally,
at least one of the one or more therapeutic agents is a drug selected from one or more of paclitaxel and related taxanes, rapamycin, sirolimus, everolimus, tacrolimus, heparin and benzalkonium heparinate.

9. The fully absorbable intraluminal device of claim 1, wherein the device is configured to achieve a device-to-luminal surface area coverage of greater than 50% once implanted into a luminal body.

10. A method of fabricating a fully absorbable intraluminal device, the method comprising:
forming a wire into a radially expandable tubular wire form (200) having a series of continuous and physically uncoupled sinusoidal, planar waveform segments (202), the wire form (200) comprising more than 50% by weight of one or more metals selected from magnesium, iron, zinc, calcium and manganese and being substantially free of rare earth metals;
coating the formed tubular wire form (200) with a polymer surface coating having a linear polyester high polymer selected from one or more of polylactic acid, polyglycolic acid, polydioxanone, polytrimethylenecarbonate and copolymers and blends thereof; and
bonding at least a portion of an expandable polymeric mesh sleeve (300) to the polymer surface coating to form a mechanical coupling with the tubular wire form (200), the expandable polymeric mesh sleeve (300) having a surface with a plurality of apertures (302) that are configured to provide strain relief and improve expandability of the sleeve (300);
wherein the device is fully absorbable, yet able to maintain a structural integrity sufficient for acutely holding a luminal body open.

11. The method of claim 10, wherein the device is fully absorbable within 30 to 365 days after being implanted into the luminal body.

12. The method of claim 10, further comprising laser cutting one or more patterns in the expandable polymeric mesh sleeve (300) to form the plurality of apertures (302) prior to bonding the sleeve to the surface of the tubular wire form (200).

13. The method of claim 10, wherein the magnesium alloy wire form (200) comprises between 1% and 25% by weight lithium.

14. The method of claim 10, further comprising coating the device with one or more therapeutic agents selected from an anti-restenotic agent, an anti-stenotic agent, an antiproliferative agent, an immunomodulator, an antithrombotic, an antioxidant, estrogen, a growth factor inhibitor, an antisense oligonucleotide, a collagen inhibitor, a chemotherapeutic agent and combinations thereof;
wherein, optionally,
at least one of the one or more therapeutic agents is a drug selected from one or more of paclitaxel and related taxanes, rapamycin, sirolimus, everolimus, tacrolimus, heparin and benzalkonium heparinate.

15. The method of claim 10, wherein the expandable polymeric mesh sleeve (300) is comprised of a linear polyester high polymer selected from one or more of polylactic acid, polyglycolic acid, polydioxanone, polytrimethylenecarbonate and copolymers and blends thereof.

## Patentansprüche

1. Vollständig absorbierbare intraluminale Vorrichtung, umfassend:
eine schraubenförmige rohrförmige Magnesiumlegierungsstruktur (200) umfassend eine Reihe von kontinuierlichen und in Längsrichtung ungekoppelten sinusförmigen, flach wellenförmigen Segmenten (202), wobei die rohrförmige Magnesiumlegierungsstruktur (200) eine Polymer-Oberflächenbeschichtung aufweist und im Wesentlichen frei von Seltenerdmetallen ist; und
eine ausdehnbare polymere Netzhülse (300), die zumindest teilweise mit der Polymer-Oberflächenbeschichtung verbunden ist, wobei die ausdehnbare polymere Netzhülse (300) dazu eingerichtet ist, eine mechanische Kopplung mit der Magnesiumlegierungsstruktur (200) zu bilden, wobei die ausdehnbare polymere Netzhülse (300) eine Vielzahl von Öffnungen (302) umfasst, die dazu eingerichtet sind, Spannungsentlastung bereitzustellen und die Ausdehnbarkeit der Hülse (300) zu verbessern.

2. Vollständig absorbierbare intraluminale Vorrichtung nach Anspruch 1, wobei die Magnesiumlegierungsstruktur (200) eine Magnesiumlegierungsdrahtform umfasst.

3. Vollständig absorbierbare intraluminale Vorrichtung nach Anspruch 1, wobei die Vorrichtung innerhalb von 30 bis 365 Tagen, nachdem sie in einen luminalen Körper implantiert ist, vollständig absorbierbar ist, jedoch in der Lage ist, eine strukturelle Integrität aufrechtzuerhalten, die ausreichend ist, um den luminalen Körper offen zu halten.

4. Vollständig absorbierbare intraluminale Vorrichtung nach Anspruch 1, wobei die Magnesiumlegierungsstruktur (200) eine Strebendicke zwischen 50 Mikron (µm) und 150 Mikron (µm) aufweist.

5. Vollständig absorbierbare intraluminale Vorrichtung nach Anspruch 1, wobei die Polymer-Oberflächenbeschichtung aus einem linearen Polyester-Hochpolymer besteht, das aus einem oder mehreren von Polymilchsäure, Polyglykolsäure, Polydioxanon, Polytrimethylencarbonat und Copolymere und Mischungen davon ausgewählt ist; oder
wobei die ausdehnbare polymere Netzhülse (300) aus einem linearen Polyester-Hochpolymer besteht, das aus einem oder mehreren von Polymilchsäure, Polyglykolsäure, Polydioxanon, Polytrimethylencarbonat und Copolymere und Mischungen davon ausgewählt ist.

6. Vollständig absorbierbare intraluminale Vorrichtung nach Anspruch 1, wobei die ausdehnbare polymere Netzhülse (300) zumindest teilweise mit einer Innenfläche der Polymer-Oberflächenbeschichtung verbindbar ist, um die indirekte Verbindung mit der Magnesiumlegierungsstruktur (200) zu bilden; oder
wobei die ausdehnbare polymere Netzhülse (300) zumindest teilweise mit einer Außenfläche der Polymer-Oberflächenbeschichtung verbindbar ist, um die indirekte Verbindung mit der Magnesiumlegierungsstruktur (200) zu bilden.

7. Vollständig absorbierbare intraluminale Vorrichtung nach Anspruch 1, wobei die Vorrichtung dazu eingerichtet ist, in Verbindung mit einem oder mehreren separaten, nicht absorbierbaren strahlenundurchlässigen Merkmalen zu funktionieren.

8. Vollständig absorbierbare intraluminale Vorrichtung nach Anspruch 1, ferner umfassend ein oder mehrere therapeutische Mittel, die aus einem anti-restenotischen Mittel, einem anti-stenotischen Mittel, einem antiproliferativen Mittel, einem Immunomodulator, einem Antithrombotikum, einem Antioxidans, Östrogen, einem Wachstumsfaktor-Inhibitor, einem Antisense-Oligonukleotid, einem Kollagen-Inhibitor, einem chemotherapeutischen Mittel und Kombinationen davon ausgewählt sind;
wobei, optional,
mindestens eines des einen oder der mehreren therapeutischen Mitteln ein Droge ist, die aus einem oder mehreren von Paclitaxel und verwandten Taxanen, Rapamycin, Sirolimus, Everolimus, Tacrolimus, Heparin und Benzalkoniumheparinat ausgewählt ist.

9. Vollständig absorbierbare intraluminale Vorrichtung nach Anspruch 1, wobei die Vorrichtung dazu eingerichtet ist, eine Vorrichtung-zu-Luminalflächen-Abdeckung von mehr als 50% erreichen, sobald sie in einen Luminalkörper implantiert ist.

10. Verfahren zur Herstellung einer vollständig absorbierbaren intraluminalen Vorrichtung, wobei das Verfahren Folgendes umfasst:
Ausbilden eines Drahtes in eine radial ausdehnbare rohrförmige Drahtform (200) aufweisend eine Reihe von kontinuierlichen und physisch ungekoppelten sinusförmigen, flach wellenförmigen Segmenten (202), wobei die Drahtform (200) mehr als 50 Gew.-% von einem oder mehreren Metallen umfasst, die aus Magnesium, Eisen, Zink, Kalzium und Mangan ausgewählt sind, und im Wesentlichen frei von Seltenerdmetallen ist;
Beschichten der ausgebildeten rohrförmigen Drahtform (200) mit einer Polymer-Oberflächenbeschichtung aufweisend ein linearen Polyester-Hochpolymer, das aus einem oder mehreren von Polymilchsäure, Polyglykolsäure, Polydioxanon, Polytrimethylencarbonat und Copolymere und Mischungen davon ausgewählt ist; und
Verbinden von mindestens einem Teil einer ausdehnbaren polymeren Netzhülse (300) mit der Polymer-Oberflächenbeschichtung, um eine mechanische Kopplung mit der rohrförmigen Drahtform (200) zu bilden, wobei die ausdehnbare polymere Netzhülse (300) eine Oberfläche mit einer Vielzahl von Öffnungen (302) aufweist, die dazu eingerichtet sind, Spannungsentlastung bereitzustellen und die Ausdehnbarkeit der Hülse (300) zu verbessern;
wobei die Vorrichtung vollständig absorbierbar ist, jedoch in der Lage ist, eine strukturelle Integrität aufrechtzuerhalten, die ausreichend ist, um einen luminalen Körper akut offen zu halten.

11. Verfahren nach Anspruch 10, wobei die Vorrichtung innerhalb von 30 bis 365 Tagen, nachdem sie in den luminalen Körper implantiert ist, vollständig absorbierbar ist.

12. Verfahren nach Anspruch 10, ferner umfassend das Laserschneiden von einem oder mehreren Mustern in der ausdehnbaren polymeren Netzhülse (300), um die Vielzahl von Öffnungen (302) vor dem Verbinden der Hülse mit der Oberfläche der rohrförmigen Drahtform (200) zu bilden.

13. Verfahren nach Anspruch 10, wobei die Magnesiumlegierungs-Drahtform (200) zwischen 1 Gew.-% und 25 Gew.-% Lithium umfasst.

14. Verfahren nach Anspruch 10, ferner umfassend das Beschichten der Vorrichtung mit einem oder mehreren therapeutischen Mitteln, die aus einem anti-restenotischen Mittel, einem anti-stenotischen Mittel, einem antiproliferativen Mittel, einem Immunomodulator, einem Antithrombotikum, einem Antioxidans, Östrogen, einem Wachstumsfaktor-Inhibitor, einem Antisense-Oligonukleotid, einem Kollagen-Inhibitor, einem chemotherapeutischen Mittel und Kombinationen davon ausgewählt sind;
wobei, optional,
mindestens eines des einen oder der mehreren therapeutischen Mitteln ein Droge ist, die aus einem oder mehreren von Paclitaxel und verwandten Taxanen, Rapamycin, Sirolimus, Everolimus, Tacrolimus, Heparin und Benzalkoniumheparinat ausgewählt ist.

15. Verfahren nach Anspruch 10, wobei die ausdehnbare polymere Netzhülse (300) aus einem linearen Polyester-Hochpolymer besteht, das aus einem oder mehreren von Polymilchsäure, Polyglykolsäure, Polydioxanon, Polytrimethylencarbonat und Copolymere und Mischungen davon ausgewählt ist.

## Revendications

1. Dispositif intraluminal entièrement absorbable, comprenant :
une structure tubulaire hélicoïdale en alliage de magnésium (200) comportant une série de segments de forme d'onde sinusoïdale plans, continus et non couplés dans le sens longitudinal (202), la structure d'alliage de magnésium tubulaire (200) ayant un revêtement de surface en polymère et étant essentiellement exempte de métaux du groupe des terres rares ; et
un manchon en grille polymérique (300) extensible au moins partiellement collé au revêtement de surface en polymère, le manchon en grille polymérique extensible (300) étant configuré pour former un accouplement mécanique avec la structure en alliage de magnésium (200), le manchon en grille polymérique extensible (300) comportant une pluralité d'ouvertures (302) qui sont configurées pour assurer la libération des contraintes et améliorer l'extensibilité du manchon (300).

2. Dispositif intraluminal entièrement absorbable selon la revendication 1, dans lequel la structure en alliage de magnésium (200) comprend une forme en fil d'alliage de magnésium.

3. Dispositif intraluminal entièrement absorbable selon la revendication 1, le dispositif étant entièrement absorbable dans les 30 à 365 jours après avoir été implanté dans un corps luminal, mais capable de maintenir une intégrité structurale suffisante pour garder le corps luminal ouvert de façon aiguë.

4. Dispositif intraluminal entièrement absorbable selon la revendication 1, dans lequel la structure en alliage de magnésium (200) a une épaisseur de tige entre 50 microns (µm) et 150 microns (µm).

5. Dispositif intraluminal entièrement absorbable selon la revendication 1, dans lequel le revêtement de surface en polymère est constitué d'un haut polymère de polyester linéaire sélectionné entre un ou plusieurs de : acide polylactique, acide polyglycolique, polydioxanone, polytriméthylènecarbonate et des copolymères et des mélanges de ceux-ci ;
ou
dans lequel le manchon en grille polymérique extensible (300) est constitué d'un haut polymère de polyester linéaire sélectionné entre un ou plusieurs de : acide polylactique, acide polyglycolique, polydioxanone, polytriméthylènecarbonate et des copolymères et des mélanges de ceux-ci.

6. Dispositif intraluminal entièrement absorbable selon la revendication 1, dans lequel le manchon en grille polymérique extensible (300) peut être au moins partiellement collé à une surface intérieure du revêtement de surface en polymère pour former la liaison indirecte avec la structure en alliage de magnésium (200) ;
ou
dans lequel le manchon en grille polymérique extensible (300) peut être au moins partiellement collé à une surface extérieure du revêtement de surface en polymère pour former la liaison indirecte avec la structure en alliage de magnésium (200).

7. Dispositif intraluminal entièrement absorbable selon la revendication 1, le dispositif étant configuré pour fonctionner conjointement avec un ou plusieurs éléments discrets non absorbables, opaques aux rayons X.

8. Dispositif intraluminal entièrement absorbable selon la revendication 1, comprenant en outre un ou plusieurs agents thérapeutiques sélectionnés entre : un agent anti-resténotique, un agent anti-sténotique, un agent antiprolifératif, un agent immuno-modulateur, un antithrombotique, un antioxydant, estrogène, un inhibiteur de facteur de croissance, un oligonucléotide antisens, un inhibiteur de collagène, un agent chimiothérapeutique et des combinaisons de ceux-ci ;
dans lequel, optionnellement,
au moins l'un du ou des agents thérapeutiques est un médicament sélectionné entre un ou plusieurs de : paclitaxel et taxanes connexes, rapamycine, sirolimus, évérolimus, tacrolimus, héparine et héparinate de benzalkonium.

9. Dispositif intraluminal entièrement absorbable selon la revendication 1, le dispositif étant configuré pour atteindre une couverture dispositif-surface luminale supérieure à 50 % une fois implanté dans un corps luminal.

10. Procédé de fabrication d'un dispositif intraluminal entièrement absorbable, le procédé comprenant les étapes consistant à :
former un fil métallique pour obtenir une forme en fil métallique tubulaire extensible radialement (200) ayant une série de segments de forme d'onde sinusoïdale plans continus et non couplés physiquement (202), la forme en fil métallique (200) comprenant plus de 50% en poids d'un ou plusieurs métaux sélectionnés entre : magnésium, fer, zinc, calcium et manganèse et étant essentiellement exempte de métaux du groupe des terres rares ;
enduire la forme en fil métallique tubulaire formée (200) d'un revêtement de surface en polymère ayant un haut polymère de polyester linéaire sélectionné entre un ou plusieurs de : acide polylactique, acide polyglycolique, polydioxanone, polytriméthylènecarbonate et des copolymères et mélanges de ceux-ci ; et
coller au moins une partie du manchon de grille polymérique extensible (300) au revêtement de surface en polymère pour former un couplage mécanique avec la forme en fil métallique tubulaire (200), le manchon de grille polymérique extensible (300) ayant une surface comportant une pluralité d'ouvertures (302) qui sont configurées pour assurer la libération des contraintes et améliorer l'extensibilité du manchon (300);
dans lequel le dispositif est entièrement absorbable, mais capable de maintenir une intégrité structurale suffisante pour garder un corps luminal ouvert de façon aiguë.

11. Procédé selon la revendication 10, dans lequel le dispositif est entièrement absorbable dans les 30 à 365 jours après avoir été implanté dans le corps luminal.

12. Procédé selon la revendication 10, comprenant en outre le découpage au laser d'un ou plusieurs motifs dans le manchon en grille polymérique extensible (300) pour former la pluralité d'ouvertures (302) avant le collage du manchon à la surface de la forme en fil métallique tubulaire (200).

13. Procédé selon la revendication 10, dans lequel la forme en fil d'alliage de magnésium (200) comprend entre 1 % et 25 % en poids de lithium.

14. Procédé selon la revendication 10, comprenant en outre le revêtement du dispositif avec un ou plusieurs agents thérapeutiques sélectionnés entre : un agent anti-resténotique, un agent anti-sténotique, un agent antiprolifératif, un agent immuno-modulateur, un antithrombotique, un antioxydant, estrogène, un inhibiteur de facteur de croissance, un oligonucléotide antisens, un inhibiteur de collagène, un agent chimiothérapeutique et des combinaisons de ceux-ci ;
dans lequel, optionnellement,
au moins l'un du ou des agents thérapeutiques est un médicament sélectionné entre un ou plusieurs de : paclitaxel et taxanes connexes, rapamycine, sirolimus, évérolimus, tacrolimus, héparine et héparinate de benzalkonium.

15. Procédé selon la revendication 10, dans lequel le manchon de grille polymérique extensible (300) est constitué d'un haut polymère de polyester linéaire sélectionné entre un ou plusieurs de : acide polylactique, acide polyglycolique, polydioxanone, polytriméthylènecarbonate et des copolymères et mélanges de ceux-ci.
